# EUROPÄISCHE PATENTANMELDUNG

(11) **EP 1 792 611 A1**
(43) Veröffentlichungstag der Anmeldung: **06.06.2007**
(21) Anmeldenummer: 06021200.8
(22) Anmeldetag: 12.07.2001
(51) Int. Cl.: A61K 9/70, A61K 38/55

(54) **Matrixkontrolliertes transdermales System für stabile Derivate der ACE-Hemmer**

(30) Priorität: 12.07.2000 DE 10033855
(62) Teilanmeldung aus: 01960496.6
(71) Anmelder: HEXAL AG, 83607 Holzkirchen (DE)
(72) Erfinder: Klokkers, Karin, 83607 Holzkirchen (DE); Kramer, Kai-Thomas, 83607 Holzkirchen (DE); Fischer, Wilfried, 83607 Holzkirchen (DE); Sendl-Lang, Anna, 83607 Holzkirchen (DE)
(74) Vertreter: Forstmeyer, Dietmar

(57) **Zusammenfassung**

Die Erfindung betrifft ein matrixkontrolliertes transdermales therapeutisches System, umfassend (i) eine wirkstoffunabhängige Deckschicht, (ii) eine selbstklebende Matrixschicht oder mehrere Matrixschichten, von denen mindestens die bei Applikation des Systems freiliegende Matrixschicht selbstklebend ist, oder eine oder mehrere Matrixschichten, deren von der Deckschicht abgewandte und zur Haftung am Applikationsort vorgesehene Fläche mit einem Haftkleber beschichtet ist, wobei die Matrixschicht(en) mindestens einen ACE-Hemmer (Angiotensin Converting Enzyme-Hemmer) in Form einer Dicarbonsäure enthält, die zu einem Derivat, ausgewählt aus der folgenden Gruppe, derivatisiert ist:
Diester,
mit Säure(n) erhältliches Mono-salz,
und (iii) eine abziehbare Schutzschicht.

## Beschreibung

Die Erfindung betrifft ein stabiles, wirkstoffhaltiges transdermales therapeutisches System zur Anwendung von stabilen Derivaten solcher ACE-Hemmern, deren Metaboliten eine Dicarbonsäure darstellen. Stabile Derivate dieser ACE-Hemmer werden durch Salzbildung oder Veresterung der Dicarbonsäuren erzielt.

Die Langzeittherapie der Hypertonie mit Angiotensin Converting Enzyme-Hemmern (ACE-Hemmer) nimmt einen immer breiteren Raum ein.
ACE-Hemmer sind bei guter Verträglichkeit für ihre zuverlässige Wirksamkeit bekannt. Bisher auf dem Markt erhältlich sind nur orale Darreichungsformen der ACE-Hemmer, wie Tabletten oder Kapseln. Der Nachteil oraler Darreichungsformen besteht darin, daß der Patient jeden Tag mindestens eine Tablette oder Kapsel schlucken muß und der Blut-Plasmaspiegel immer gewissen Schwankungen unterworfen ist. Ein gleichbleibender Plasmaspiegel ist mit oralen Darreichungsformen kaum zu gewährleisten.

Die transdermale Applikation dagegen bietet für ACE-Hemmer eine Reihe von Vorteilen:
die Haut ist unbegrenzt zugänglich,
es erfolgt kein Milieuwechsel wie bei der peroralen Applikation,
die Handhabung ist einfach und bequem,
es genügt normalerweise eine einmalige Gabe statt mehrfacher täglicher Gaben,
die Patienten-Compliance ist wesentlich besser,
es ist eine kontinuierliche Langzeittherapie möglich,
die Freisetzung des Wirkstoffes erfolgt annähernd gemäß einer Kinetik 0-ter Ordnung, eine Therapie kann schneller unterbrochen werden,
es wird ein konstanter Plasmaspiegel über längere Zeit sichergestellt,
ein anfangs zu hoher Plasmaspiegel, wie bei intravenöser Applikation wird vermieden und
aufgrund der Umgehung der 1. Passage bedarf es teilweise einer niedrigeren Dosierung als bei der oralen Gabe, wodurch eine geringere Nebenwirkungsrate auftritt und
die Gefahr von Über- oder Unterdosierung geringer ist.
Aus WO-A1-93/23019 ist bereits ein transdermales Reservoirsystem mit einem Gehalt an einem ACE-Hemmer und
a) einer undurchlässigen Abdeckschicht (Backing Layer)
b) einem schichtartigen Element mit Hohlraum,
c) einem die Wirkstoffabgabe steuernden Mittel (claim 1) und
d) einer abziehbaren Deckschicht (Release Liner) auf Papierbasis (Seite 12 Zeile 7/8) bekannt.

Transdermale Systeme mit einem Gehalt an einem ACE-Hemmer werden ferner in EP-A-0 439 430 (Reservoir-TTS) und EP-A-0 468 875 (Matrix-TTS) beschrieben, wobei nach EP-A-0 468 875 Silikon-Elastomere als Matrixmaterial verwendet werden. EP-A-452 837 beschreibt eine Matrix für Pflaster, das u.a. ACE-Hemmer als Wirkstoffe enthält. Allerdings werden als mögliche ACE-Hemmer konkret Delaprilhydrochlorid, Enalaprilmaleat, Captopril, Alacepril und (R)-3-[(S)-1-Carboxy-5-(4-piperidyl)-pentyl]-amino-4-oxo-2,3,4,5-tetrahydro-1,5-benzothiazepin-5-Essigsäure genannt. WO 96/29999 beschreibt ein TTS mit einer Matrix auf Basis von Polyisobutylen oder Butylkautschuk mit einem Gehalt an Trandolapril und/ oder Ramipril.

Es hat sich nun gezeigt, daß für ein Matrix-TTS von bestimmten unstabilisierten ACE-Hemmern die Stabilität des Wirkstoffes im Pflaster den Anforderungen nicht genügt.

Die Zersetzung des ACE-Hemmers in der Matrix erfolgt in einem derart großen Ausmaß, daß schon nach kurzer Lagerzeit der Gehalt an den Zersetzungsprodukten so hoch ist, daß die Zulässigkeitsgrenze an Abbauprodukten weit überschritten wird. Auch kann mit unstabilisierten ACE-Hemmern keine ausreichende in-vitro-Permeation durch die Haut erreicht werden.

Die Aufgabe der vorliegenden Erfindung ist die Bereitstellung eines Matrix-TTS mit einem Gehalt an stabilem Derivat von ACE-Hemmern, dessen Stabilität bezüglich des Abbaus des Wirkstoff den gesetzlichen Anforderungen entspricht und dessen in-vitro-Hautpermeation deutlich gesteigert ist. Der Wirkstoffgehalt soll über einen längeren Zeitraum stabil sein und praktisch keinen Zersetzungsvorgängen unterworfen sein. Die Klebkraft des Matrix-Pflasters soll für eine Tragedauer von mind. 3 Tage ausreichend sein.

Überraschenderweise wurde nun gefunden, daß die Salze der aktiven Metaboliten (= Dicarbonsäure) von ACE-Hemmern, die durch Umsetzung der Dicarbonsäure mit starken Säuren (1:1) oder Basen (1:2) gebildet werden, einerseits weitgehend stabil gegen Zersetzung sind und andererseits hervorragende Hautpermeation aufweisen. Die Salze der aktiven Metaboliten können dabei in der Matrixschicht in-situ gebildet werden.

Eine Stabilisierung der ACE-Hemmer kann erstaunlicherweise auch durch eine zweifache Veresterung ihrer Metaboliten erreicht werden. Durch die damit verbundene Erhöhung der Lipophilie des ACE-Hemmers läßt sich eine ausgezeichnete Hautpermeation erzielen.

In manchen Fällen sind die stabilen Derivate der ACE-Hemmer derart hygroskopisch, daß sich das Pflaster schon nach kurzer Zeit von der Haut ablöst. Diese Schwierigkeit wurde gelöst, indem über das eigentliche Matrixpflaster, bestehend aus einer für den Wirkstoff undurchlässigen Deckschicht, einer wirkstoffhaltigen, selbstklebenden Matrix und einer abziehbaren Schutzschicht, noch ein "Overtape" aufgebracht wurde. Das "Overtape" kann das eigentliche Matrixpflaster an allen Seiten überragen. Unter "Overtape" versteht man einen Verbund aus einer Deckschicht und einer Kleberschicht.

Die der Erfindung zugrundeliegende Aufgabe wird nun durch ein matrixkontrolliertes transdermales therapeutisches System mit einem Gehalt an mindestens einem stabilen Derivat eines ACE-Hemmers gelöst. Die Klebkraft des Pflasters und dessen Trageeigenschaften können gegebenenfalls durch das Aufbringen eines "Overtapes" entscheidend verbessert werden.

Das erfindungsgemäße transdermale therapeutische System kann aus einer für den Wirkstoff undurchlässigen Deckschicht (5), aus einer oder mehreren den Wirkstoff und/oder fakultative Permeationsförderer enthaltenden, selbstklebenden Matrixschicht(en) (6) oder einer oder mehreren Matrixschicht(en) (9), die mit einem Haftkleber (8) beschichtet sind und einer abziehbaren Schutzschicht (7) bestehen. Im Falle hygroskopischer, stabilisierter ACE-Hemmer wird ein "Overtape" (1), das aus einem Verbund aus einer Deckschicht (3) und einer Haftklebeschicht (4) zusammengesetzt ist, verwendet. Das "Overtape" (1) kann das übrige System (2) an allen Seiten überragen.

In dem erfindungsgemäßen transdermalen therapeutischen System kann mindestens ein stabiles Salz der aktiven Metaboliten (= Dicarbonsäure) eines ACE-Hemmers, das auf einer Reaktion einer sauer oder alkalisch reagierenden Verbindung mit der Dicarbonsäure basiert, verwendet werden.
Als sauer reagierende Verbindungen kommen in Frage: anorganische Säuren, wie z. B. Salzsäure, Bromwasserstoffsäure, Iodwasserstoffsäure, Salpetersäure, Schwefelsäure, Phosphorsäure, organische Carbonsäuren, wie z. B. Salicylsäure, Maleinsäure, Adipinsäure, Sorbinsäure, Malonsäure, 1,4-Butandisäure, Äpfelsäure, Pivalinsäure, Bernsteinsäure, Nicotinsäure, Isonicotinsäure, Furan-2-carbonsäure, Dichloressigsäure, Benzoesäure, Fettsäuren, wie z. B. Laurinsäure, Myristylsäure, Ölsäure, aliphatische Sulfonsäuren, wie z.B. Methan-, Ethan-, Propan-, Isopropan-, Butan-, Isobutan-, Pentan-, Isopentan-, Hexan-, Heptan-, Octan-, Nonan-, Decan-, Undecan-, Dodecansulfonsäure oder aromatische Sulfonsäuren, wie z.B. Toluol- oder Benzolsulfonsäure, insbesondere Methan-, Toluol- oder Benzolsulfonsäure. Die bevorzugt verwendete Säure ist die Methansulfonsäure.
Als alkalisch reagierende Verbindungen kommen in Frage: Natrium-, Kalium-, Magnesium-, Calcium-, Aluminiumhydroxid, alkalische Ammoniumsalze, organische Amine wie Ethylendiamin, Ethylamin, Diethylamin, Dipropylamin, Diisopropylamin, Tripropylamin, Trihexylamin, Tridodecylamin, Dicyclohexylamin, Ethanolamin, Diethanolamin, Triethanolamin, Triisopropanolamin, 1-Amino-2-propanol, 2-Amino-2-methyl-1-propanol, Oleylamin, hetereocyclische Amine wie N-Methylpiperazin oder 1-(2-Hydroxyethyl)pyrrolidin. Die bevorzugt verwendete Base ist Natriumhydroxid.

Es können die stabilen Salze der aktiven Metabolite (= Dicarbonsäure) der ACE-Hemmer in der Matrix in-situ gebildet werden, indem die entsprechenden alkalisch bzw. sauer reagierenden Verbindungen und die Dicarbonsäuren zusammen in die Matrix eingearbeitet werden. Die stabilen Salze der Metaboliten der ACE-Hemmer können aber auch direkt in die Matrix eingebracht werden.

In einer alternativen Ausführungsform des erfindungsgemäßen transdermalen therapeutischen Systems kann mindestens ein stabiler zweifacher Ester eines ACE-Hemmer-Metaboliten verwendet werden. Für den Alkylrest des Esters kommen in Frage die Methyl-, Ethyl-, Propyl-, Isopropyl-, Butyl-, tert.-Butyl-, Pentyl-, Hexyl-, Heptyl-, Octyl-, Nonan-, Decangruppe und ihre Isomeren. Bevorzugt wird der Ethylester.

Das erfindungsgemäße transdermale therapeutische System kann als Wirkstoff die stabilisierten Formen solcher ACE-Hemmer enthalten, deren aktive Metaboliten eine Dicarbonsäure darstellen. Beispiele sind Imidapril, Fosinopril, Moexipril, Perindopril, Ramipril, Spirapril, Cilazapril, Benazepril und/ oder Trandolapril. Bevorzugt werden die Monosulfonsäuresalze, die Dinatriumsalze sowie die Diester von Trandolaprilat und/ oder Ramiprilat als Wirkstoffkomponenten verwendet.

Der Gehalt an ACE-Hemmern kann 2-25 Gew.%, insbesondere 10 -15 Gew.%, bezogen auf das Matrixgewicht, betragen.

Der Gehalt an Säure oder Base ist äquimolar zum Metaboliten (= Dicarbonsäure) des ACE-Hemmers, der an Base doppelt so groß ist, und hängt somit vom Molekulargewicht der Dicarbonsäure ab.

Als undurchlässige Deckschicht kommen Folien aus Acetal, Acrylat, Acrylonitril-Butadien-Styrol, Acrylonitril (Methyl Methacrylat) Copolymer, Acrylonitril Copolymer, Ethylen Ethyl Acrylat, Ethylen Methyl Acrylat, Ethylen Vinyl Acetat, Ethylen Vinyl Acetat Copolymer, Ethylen Vinylalkohol Polymer, Ionomere, Nylon (Polyamid), Nylon (Polyamid) Copolymer, Polybutylen, Polycarbonat , Polyester, Polyethylenterephthalat, thermoplastisches Polyester Copolymer, Polyethylen Copolymer (high density), Polyethylen (high-molecular-weight, high-density), Polyethylen (intermediate-molecular-weight, high-density), Polyethylen (linear low density), Polyethylen (low density), Polyethylen (medium density), Polyethylenoxid, Polyimid, Polypropylen, Polypropylen (coated), Polypropylen (oriented), Polystyrol, Polyurethan, Polyvinylacetat, Polyvinylchlorid, Polyvinylidenchlorid und/ oder Styrol- Acrylonitril in Frage, die bei Bedarf metallisiert oder pigmentiert werden können. Als für den Wirkstoff undurchlässige Deckschicht wird Polyurethan bevorzugt.

Als Deckschicht für das Overtape kommen mikroperforierte Folien aus oben genannten Materialien und Membranen aus Polyurethan, Polyethylen, Polypropylen, Polyester, Coextrudate aus Ethylvinylalkohol/Polyethylen oder Polyethylen/Polypropylen in Frage. Als Deckschicht für das Overtape werden Polyurethan Ester und Polyurethan Ether bevorzugt.

Für die Haftklebeschicht insbesondere des Overtapes kann man ein druckempfindliches Klebemittel beispielsweise auf Polyurethanbasis, Polyisobutylenbasis, Polyvinyletherbasis, Polyacrylatbasis oder ein Gemisch aus diesen wählen. Bevorzugt werden Kleber auf Acrylat- und Polyisobutylenbasis verwendet.

Bei den Klebemitteln auf Polyacrylatbasis kann es sich um ein beliebiges Homopolymer, Copolymer oder Terpolymer, bestehend aus verschiedenen Acrylsäurederivaten handeln.

So können die Polyacrylate Polymere eines oder mehrerer Monomere von Acrylsäuren und anderen copolymerisierbaren Monomeren sein. Außerdem können die Polyacrylate Copolymere von Alkylacrylaten und/ oder -methacrylaten und/ oder copolymerisierbaren sekundären Monomeren oder Monomeren mit funktionellen Gruppen umfassen. Verändert man den Betrag jeder Sorte, die als Monomer hinzugefügt ist, können die kohäsiven Eigenschaften der daraus resultierenden Acrylatpolymere verändert werden. Im allgemeinen besteht das Acrylatpolymer aus mindestens 50 Gew.-% eines Acrylat-, Methacrylat-, Alkylacrylat- oder Alkylmethacrylat-Monomers, 0 bis 20 % eines funktionellen Monomers, copolymerisierbar mit Acrylat, und 0 bis 50 % eines anderen Monomeren.

Im folgenden sind verschiedene Acrylatmonomere, wie z.B. Acrylsäure, Methacrylsäure, Butylacrylat, Butylmethacrylat, Hexylacrylat, Hexylmethacrylat, Isooctylacrylat, Isooctylmethacrylat, Glycidylmethacrylat, 2-Hydroxyethylacrylat, Methylacrylat, Methylmethacrylat, 2-Ethylhexylacrylat, 2-Ethylhexylmethacrylat, Decylacrylat, Decylmethacrylat, Dodecylacrylat, Dodecylmethacrylat, Tridecylacrylat und Tridecylmethacrylat aufgeführt, die alleine oder in Mischungen polymerisiert sein können.

Zusätzlich können funktionelle Monomere, die mit den oben genannten Acrylaten, copolymerisierbar sind, wie beispielsweise Acrylsäure, Methacrylsäure, Maleinsäure, Maleinanhydrid, Hydroxyethylacrylat, Vinylacetat, Hydroxypropylacrylat, Acrylamid, Dimethylacrylamid, Acrylnitril, Dimethylaminoethylacrylat, Dimethylaminoethylmethacrylat, tert.-Butylaminoethylacrylat, tert.-Butylaminoethylmethacrylat, Methoxyethylacrylat und Methoxyethylmethacrylat, zur Copolymerisierung eingesetzt werden.

Weitere Einzelheiten und Beispiele für druckempfindliche Acrylate, welche für die Erfindung geeignet sind, sind in Satas Handbook of Pressure Sensitive Adhesive Technology "Acrylic Adhesives", 2nd ed., pp. 396-456 (D. Satas, ed.), Van Nostrand Reinhold, New York (1989) beschrieben.

Der Gehalt an Klebemitteln der selbstklebenden Matrix kann 50 - 90 Gew.%, insbesondere 70 - 80 Gew. %, bezogen auf das Matrixgewicht, betragen.

Für die Matrix werden die medizinisch üblichen Matrixbildner wie Polyacrylat, Polyisobutylen, Kautschuk, kautschukähnliche synthetische Homo-, Co- oder Blockpolymere, Styrol/ Butadien- Copolymerisat oder ein Gemisch aus diesen, wie sie im Stand der Technik vorgesehen werden, verwendet. Bevorzugt wird eine selbstklebende Matrix aus Polyacrylat und/ oder Polyisobutylen verwendet, wobei Matrixbildner und Klebemittel eins sind.

Für die abziehbare Schutzschicht kommen Polyester, Polyethylen, Polypropylen, Polysiloxan, Polyacrylat, Ethylenvinylacetat, Polyurethan, Polyisobuten oder Papier, meistens mit Silikon- und /oder Polyethylen beschichtet, oder ein Gemisch aus diesen in Betracht.

Als Permeationsförderer lassen sich gegebenenfalls gesättigte und/ oder ungesättigte Fettalkohole mit jeweils 8-18 C- Atomen; Teebaumöl; gesättigte und/ oder ungesättigte cyclische Ketone; Alkyl- Methylsulfoxide; gesättigte und/ oder ungesättigte Fettsäuren mit jeweils 8-18 C- Atomen; deren Ester und Salze; natürliches Vitamin E; synthetisches Vitamin E und/ oder Vitamin E- Derivate; Sorbitanfettsäureester und ethoxylierte Sorbitanfettsäureester; Azone (Laurocapram); 1-Alkylpyrrolidon; Blockcopolymere von Polyethylenglykol und Dimethylsiloxan mit kationischer Gruppe an einem Ende; Polyoxyethylen-10-stearylether; Gemisch aus Polyoxyethylen-10-stearylether und Glyceryldilaurat; Dodecyl-2-(N,N-dimethylamino)-propanoltetradecanoat und/ oder Dodecyl-2-(N,N-dimethylamino)-propianat; N-Acetylprolinatester mit > 8 C-Atomen; nichtionische Tenside, z.B. Laurylether, Ester von Polyoxyethylen; Dimethyl(arylimino)sulfuran; Gemisch aus Ölsäureanaloga und Propylenglykol; Gemisch aus Padimat O, Oktylsalicylat, Isopropylmyristat, Isopropylpalmitat, Oktylmethoxycinnimat, Laurocapram; hochdisperses Siliziumdioxid (Aerosil^{®}); Polyoxyethylen-7-glycerol-monococoat (Cetiol^{®} HE); 2-Octyldodecanol (Eutanol^{®} G) oder ein Gemisch aus verschiedenen Einzelkomponenten verwenden. Es werden in dem erfindungsgemäßen transdermalen therapeutischen System hochdisperses Siliziumdioxid (Aerosil^{®}) und/oder Polyoxyethylen-7-glycerol-monococoat (Cetiol^{®} HE) und/ oder 2-Octyldodecanol (Eutanol^{®} G) als fakultative(r) Permeationsförderer bevorzugt.

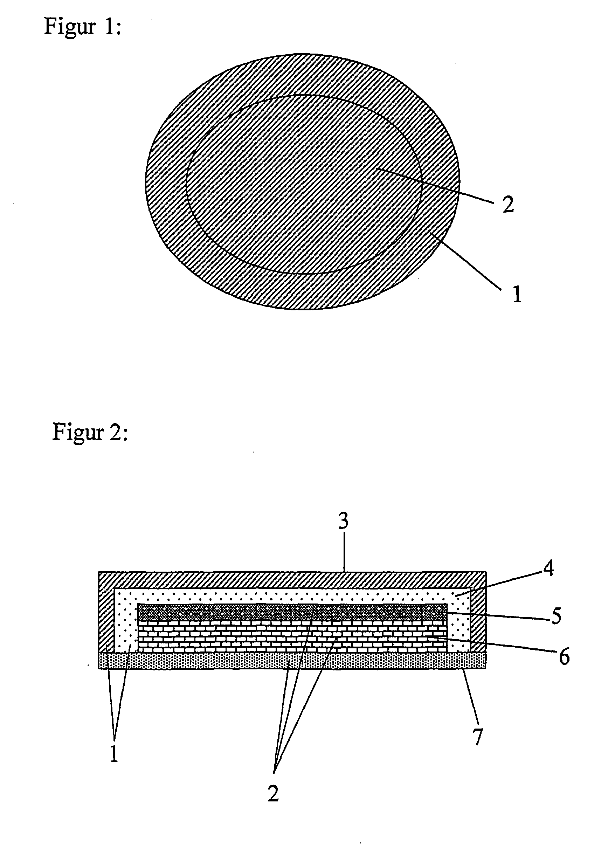

Figur 1 zeigt die Aufsicht von oben auf das transdermale therapeutische System wobei (1) das "Overtape" und (2) das übrige System darstellt.

Figur 2 zeigt das erfindungsgemäße transdermale therapeutische System mit einer selbstklebenden Matrix im Querschnitt. Die oberste Schicht stellt die für den Wirkstoff undurchlässige Deckschicht (3) dar. Darunter befindet sich eine Haftklebeschicht (4). Diese beiden Schichten bilden das "Overtape" (1). Die nächste Schicht ist abermals eine für den Wirkstoff undurchlässige Deckschicht (5). Das Material dieser Deckschicht (5) kann gleich oder verschieden zu dem Material der ersten Deckschicht (3) sein. Dann folgt die selbstklebende Matrixschicht (6), die den Wirkstoff und fakultative Permeationshemmer enthält. Das Matrixbildner ist in diesem Fall der Haftkleber. Den Abschluß bildet eine abziehbare Schutzschicht (7).

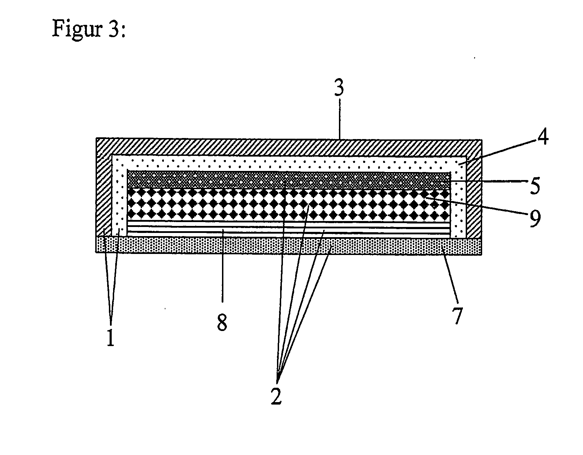

Figur 3 zeigt den Querschnitt des erfindungsgemäßen transdermalen therapeutischen Systems mit einer nicht selbstklebenden Matrixschicht (9), die mit einer separaten Haftkleberschicht (8) versehen ist.

Die Erfindung wird durch nachstehende Beispiele näher erläutert ohne aber den Erfindungsumfang damit einzuschränken.

### Beispiel 1:

Zusammensetzung einer erfindungsgemäßen selbstklebenden Matrix für ein TTS

| Inhaltsstoffe | Gehalt in Gew.% |
|---|---|
| Trandolaprildisäure | 10 |
| Methansulfonsäure | 2,4 |
| Aerosil^{®} 200 | 4 |
| Cetiol^{®} HE | 10 |
| Durotak^{®} 387-2353 | 73,6 |

Die Gewichtsprozente beziehen sich auf das Matrixgewicht.

### Herstellungsprozeß:

Aerosil^{®}, Cetiol^{®} HE, Kleber (Durotak^{®}) und Ethylacetat werden in ein geeignetes Rührgefäß eingewogen (Klebstofflösung). Parallel dazu werden in einem anderen geeigneten Rührgefäß Trandolaprildisäure und Ethylacetat eingewogen, homogenisiert, Methansulfonsäure zugegeben und so lange gerührt, bis eine klare Lösung entstanden ist (Wirkstofflösung). Dann wird die Wirkstofflösung in die Klebstofflösung überführt und homogenisiert. Diese Mischung wird auf eine Folie für die abziehbare Schutzschicht aufgetragen und im Trockenkanal getrocknet. Auf diese Matrix wird dann eine PU-Folie (z. B. Walotex 2204 ACK, 25 µm) für die wirkstoffundurchlässige Deckschicht aufgebracht. Anschließend werden die Pflaster gestanzt.

### Beispiel 2:

Zusammensetzung einer erfindungsgemäßen selbstklebenden Matrix für ein TTS

| Inhaltsstoffe | Gehalt in Gew.% |
|---|---|
| Ramiprildisäure | 10 |
| Methansulfonsäure | 2,5 |
| Aerosil^{®} 200 | 4,0 |
| Cetiol^{®} HE | 10 |
| Durotak^{®} 387-2510 | 73,5 |

Die Gewichtsprozente beziehen sich auf das Matrixgewicht.

### Herstellungsprozeß:

Aerosil^{®}, Cetiol^{®} HE, Kleber (Durotak^{®}) und Ethylacetat werden in ein geeignetes Rührgefäß eingewogen (Klebstofflösung). Parallel dazu werden in einem anderen geeigneten Rührgefäß Ramiprildisäure und Ethylacetat eingewogen, homogenisiert, Methansulfonsäure zugegeben und so lange gerührt, bis eine klare Lösung entstanden ist (Wirkstofflösung). Dann wird die Wirkstofflösung in die Klebstofflösung überführt und homogenisiert. Diese Mischung wird auf die abziehbare Schutzschicht aufgetragen und im Trockenkanal getrocknet. Die weitere Verarbeitung erfolgt wie in Beispiel 1.

### Beispiel 3:

Zusammensetzung einer erfindungsgemäßen selbstklebenden Matrix für ein TTS

| Inhaltsstoffe | Gehalt in Gew.% |
|---|---|
| Trandolaprildisäure | 10 |
| Natriumhydroxid | 1 |
| Aerosil^{®} 200 | 4 |
| Cetiol^{®} HE | 10 |
| Durotak^{®} 87-4098 | 75 |

Die Gewichtsprozente beziehen sich auf das Matrixgewicht.

### Herstellungsprozeß:

Aerosil^{®}, Cetiol^{®} HE und Ethylacetat werden in ein geeignetes Rührgefäß eingewogen und so lange gerührt, bis sich eine homogene Suspension ergeben hat. Der Kleber Durotak^{®} wird hinzugewogen (=Klebstofflösung) und diese mittels geeignetem Dispergiergerät homogenisiert und über Nacht auf einem Taumelroller gemischt. Parallel dazu werden in einem anderen geeigneten Rührgefäß Trandolaprildisäure, Natriumhydroxid und Ethylacetat eingewogen und so lange gerührt, bis eine klare Lösung entstanden ist (Wirkstofflösung). Dann wird die Wirkstofflösung in die Klebstofflösung überführt und homogenisiert. Diese Mischung wird auf eine Folie für die abziehbare Schutzschicht aufgetragen und im Trockenkanal getrocknet. Die weitere Verarbeitung erfolgt wie in Beispiel 1.

### Beispiel 4:

Zusammensetzung einer erfindungsgemäßen selbstklebenden Matrix für ein TTS

| Inhaltsstoffe | Gehalt in Gew.% |
|---|---|
| Ramiprildisäure | 10 |
| Natriumhydroxid | 1 |
| Aerosil^{®} 200 | 4 |
| Cetiol^{®} HE | 10 |
| Durotak^{®} 87-4098 | 75 |

Die Gewichtsprozente beziehen sich auf das Matrixgewicht.

### Herstellungsprozeß:

Aerosil^{®}, Cetiol ^{®} HE und Ethylacetat werden in ein geeignetes Rührgefäß eingewogen und so lange gerührt, bis sich eine homogene Suspension ergeben hat. Der Kleber Durotak^{®} wird hinzugewogen (=Klebstofflösung) und diese mittels geeignetem Dispergiergerät homogenisiert und über Nacht auf einem Taumelroller gemischt. Parallel dazu werden in einem anderen geeigneten Rührgefäß Ramiprildisäure, Natriumhydroxid und Ethylacetat eingewogen und so lange gerührt, bis eine klare Lösung entstanden ist (Wirkstofflösung). Dann wird die Wirkstofflösung in die Klebstofflösung überführt und homogenisiert. Diese Mischung wird auf eine Folie für die abziehbare Schutzschicht aufgetragen und im Trockenkanal getrocknet. Die weitere Verarbeitung erfolgt wie in Beispiel 1.

### Beispiel 5:

Zusammensetzung einer erfindungsgemäßen selbstklebenden Matrix für ein TTS

| Inhaltsstoffe | Gehalt in Gew.% |
|---|---|
| Trandolaprilethylester | 10 |
| Aerosil^{®} 200 | 4 |
| Cetiol^{®} HE | 10 |
| Durotak^{®} 387-2510 | 76 |

Die Gewichtsprozente beziehen sich auf das Matrixgewicht.

### Herstellungsprozeß:

Aerosil^{®}, Cetiol ^{®} HE, Trandolaprilethylester und Ethylacetat werden in ein geeignetes Rührgefäß eingewogen (Wirkstofflösung). Dann wird die Wirkstofflösung in die Klebstofflösung (Durotak^{®} ) überführt und homogenisiert. Diese Mischung wird auf die abziehbare Schutzschicht aufgetragen und im Trockenkanal getrocknet. Die weitere Verarbeitung erfolgt wie in Beispiel 1.

### Beispiel 6:

Zusammensetzung einer erfindungsgemäßen selbstklebenden Matrix für ein TTS

| Inhaltsstoffe | Gehalt in Gew.% |
|---|---|
| Ramiprilethylester | 10 |
| Aerosil^{®} 200 | 4 |
| Cetiol^{®} HE | 10 |
| Durotak^{®} 387-2510 | 76 |

Die Gewichtsprozente beziehen sich auf das Matrixgewicht.

### Herstellungsprozeß:

Aerosil^{®}, Cetiol^{®} HE, Ramiprilethylester und Ethylacetat werden in ein geeignetes Rührgefäß eingewogen (Wirkstofflösung). Dann wird die Wirkstofflösung in die Klebstofflösung (Durotak^{®}) überführt und homogenisiert. Diese Mischung wird auf die abziehbare Schutzschicht aufgetragen und im Trockenkanal getrocknet. Die weitere Verarbeitung erfolgt wie in Beispiel 1.

## Patentansprüche

1. Matrixkontrolliertes transdermales therapeutisches System, umfassend (i) eine wirkstoffundurchlässige Deckschicht, (ii) eine selbstklebende Matrixschicht oder mehrere Matrixschichten, von denen mindestens die bei Applikation des Systems freiliegende Matrixschicht selbstklebend ist, oder eine oder mehrere Matrixschichten, deren von der Deckschicht abgewandte und zur Haftung am Applikationsort vorgesehene Fläche mit einem Haftkleber beschichtet ist, wobei die Matrixschichten mindestens einen ACE-Hemmer (Angiotensin Converting Enzyme-Hemmer) in Form einer Dicarbonsäure enthält, die zu einem Derivat, ausgewählt aus der folgenden Gruppe, derivatisiert ist:
Diester,
mit Base(n) erhältliches Di-salz und
mit Säure(n) erhältliches Mono-salz,
und (iii) eine abziehbare Schutzschicht.

2. System nach Anspruch 1, **gekennzeichnet durch** mindestens einen ACE-Hemmer aus der Gruppe Imidapril, Fosinopril, Moexipril, Perindopril, Ramipril, Spirapril, Cilazapril, Benazepril und/oder Trandolapril in Form einer Dicarbonsäure, die zu einem Diester, einem mit Base(n) gebildeten Di-salz und/oder einem mit Säure(n) gebildeten Mono-salz derivatisiert ist.

3. System nach Anspruch 1, **gekennzeichnet durch** mindestens einen ACE-Hemmer aus der Gruppe Imidapril, Fosinopril, Moexipril, Perindopril, Ramipril, Spirapril, Cilazapril und/oder Trandolapril in Form einer Dicarbonsäure, die zu einem Diester, einem mit Base(n) gebildeten Di-salz und/oder einem mit Säure(n) gebildeten Mono-salz derivatisiert ist.

4. System nach Anspruch 2 und/oder 3, **gekennzeichnet durch** Ramipril und/oder Trandolapril, insbesondere Mono-sulfonsäuresalz oder Dinatriumsalz von Trandolaprilat und/oder Ramiprilat.

5. System nach Anspruch 2 und/oder 3, **gekennzeichnet durch** einen Ethylester des Trandolaprils und/oder Ramiprils.

6. System nach Anspruch 1, 2 und/oder 3, **dadurch gekennzeichnet, daß** der ACE-Hemmer zusätzlich zu einer ersten Estergruppe eine weitere Estergruppe aus der folgenden Gruppe trägt: Methyl-, Ethyl-, Propyl-, Isopropyl-, Butyl-, tert.-Butyl-, Pentyl-, Hexyl-, Heptyl-, Octyl-, Nonan-, Decangruppe und ihre Isomeren; wobei die erste Estergruppe frei gewählt ist, wobei die erste Estergruppe frei gewählt ist, wobei der ACE-Hemmer eine pharmazeutisch verträgliche Verbindung ist, oder wobei die erste und die zweite Estergruppe identisch sind.

7. System nach Anspruch 6, **dadurch gekennzeichnet, daß** die weitere Estergruppe eine Ethylgruppe ist.

8. System nach mindestens einem der vorhergehenden Ansprüche, **gekennzeichnet durch** ein Di-salz gemäß Anspruch 1, das mit einer Base aus der folgenden Gruppe erhältlich ist: Natrium-, Kalium-, Magnesium-, Calcium-, Aluminiumhydroxid, alkalisches Ammoniumsalz, organisches Amin, insbesondere Ethylendiamin, Ethylamin, Diethylamin, Dipropylamin, Diisopropylamin, Tripropylamin, Trihexylamin, Tridodecylamin, Dicyclohexylamin, Ethanolamin, Diethanolamin, Triethanolamin, Triisopropanolamin, 1-Amino-2-pro panol, 2-Amino-2-methyl-1-propanol und Oleylamin und hetero-cyclische Amine, insbesondere N-Methylpiperazin und 1-(2-Hydroxyethyl)pyrrolidin.

9. System nach Anspruch 8, **gekennzeichnet durch** Natriumhydroxid als Base.

10. System nach mindestens einem der Ansprüche 1 bis 7, **gekennzeichnet durch** ein Monosalz gemäß Anspruch 1, das mit einer Säure aus der folgenden Gruppe erhältlich ist: anorganische Säure, insbesondere Salzsäure, Bromwasserstoffsäure, Iodwasserstoffsäure, Salpetersäure, Schwefelsäure und Phosphorsäure, organische Carbonsäure, insbesondere Salicylsäure, Maleinsäure, Adipinsäure, Sorbinsäure, Malonsäure, 1,4-Butandisäure, Äpfelsäure, Pivalinsäure, Bernsteinsäure, Nicotinsäure, Isonicotinsäure, Furan-2-carbonsäure, Dichloressigsäure und Benzoesäure, Fettsäuren, insbesondere Laurinsäure, Myristylsäure und Ölsäure, aliphatische Sulfonsäure, insbesondere Methan-, Ethan-, Propan-, Isopropan-, Butan-, Isobutan-, Pentan-, Isopentan-, Hexan-, Heptan-, Octan-, Nonan-, Decan-, Undecan- und Dodecansulfonsäure und aromatische Sulfonsäure, insbesondere Toluol- und Benzblsulfonsäure.

11. System nach Anspruch 10, **gekennzeichnet durch** Methansulfonsäure als Säure.

12. System nach mindestens einem der vorhergehenden Ansprüche, **dadurch gekennzeichnet, daß** ACE-Hemmer
(i) vor Bildung seines Di-salzes gemäß Anspruch 1 zur Salzbildung mit Base(n) in einem molaren Verhältnis getrennt voneinander oder
(ii) vor Bildung eines Mono-salzes gemäß Anspruch 1 zur Salzbildung mit Säure(n) in equimolarem Verhältnis getrennt voneinander oder
(iii) das Di-salz gemäß Anspruch 1 oder das Mono-salz gemäß Anspruch 1
in das System eingearbeitet worden sind.

13. System nach mindestens einem der vorhergehenden Ansprüche, **gekennzeichnet durch** einen Gehalt an ACE-Hemmern(n) von 2 bis 25 Gew.-% und insbesondere 10 bis 15 Gew.-%, bezogen auf das Matrixgewicht.

14. System nach mindestens einem der vorhergehenden Ansprüche, **dadurch gekennzeichnet, daß** das System auf der Seite der Deckschicht, die der (den) Matrixschicht(en) abgewandt ist, (iv) eine Abdeckung (Overtape) aufweist,
(i) die die Deckschicht allseitig überragt und flächendeckend oder zumindest auf der die Deckschicht überragenden und in sich geschlossenen Zone mit einem Haftklebemittel versehen ist, oder
(ii) die die Deckschicht flächendeckend abdeckt, jedoch nicht überragt, und flächendeckend mit einem Haftklebemittel versehen ist.

15. System nach Anspruch 14, **dadurch gekennzeichnet, daß** die mit einem Haftklebemittel versehene Abdeckung (Overtape) die wirkstoffundurchlässige Deckschicht vollständig abdeckt oder über der Deckschicht mit einer oder mehreren Perforationen versehen ist oder ringförmig ausgebildet ist.

16. System nach mindestens einem der Ansprüche 14 oder 15, **dadurch gekennzeichnet, daß** die wirkstoffundurchlässige Deckschicht und die mit einem Haftklebemittel versehene Abdeckung wasserdampfdurchlässig sind.

17. System nach einem der vorhergehenden Ansprüche, **dadurch gekennzeichnet, daß** die wirkstoffundurchlässige Deckschicht und die mit einem Haftklebemittel versehene Abdeckung (Overtape) aus demselben Material bestehen.

18. System nach mindestens einem der vorhergehenden Ansprüche, **dadurch gekennzeichnet, daß** die Matrixschicht(en) ein oder mehrere Permeationsförderer enthalten.

19. System nach Anspruch 18, **gekennzeichnet durch** hochdisperses Siliziumdioxid, Polyoxyethylen-7-glycerol-monococoat und/oder 2-Octyldodecanol (Eutanol G) als Permeationsförderer.
